# EUROPEAN PATENT APPLICATION

(11) **EP 1 535 567 A1**
(43) Date of publication of application: **01.06.2005**
(21) Application number: 04028137.0
(22) Date of filing: 26.11.2004
(51) Int. Cl.: A61B 3/16, A61B 3/18

(54) **Ophthalmic apparatus**

(30) Priority: 28.11.2003 JP 2003400194
(71) Applicant: Nidek Co., Ltd., Gamagori-shi, Aichi-ken 443-0035 (JP)
(72) Inventor: Murakami, Yasuhisa, Toyohashi-shi Aichi-ken 441-3301 (JP)
(74) Representative: Winter, Brandl, Fürniss, Hübner, Röss, Kaiser, Polte Partnerschaft Patent- und Rechtsanwaltskanzlei

(57) **Abstract**

A multifunction ophthalmic apparatus, capable of examining different eye characteristics of an examinee's eye by a single apparatus, has a first examination part including a first examination optical system for examining a first eye characteristic, a second examination part including a second examination optical system for examining a second eye characteristic, a first image-pickup element which picks up a first image of an anterior-segment of the eye in first examination, a second image-pickup element which picks up a second image of the anterior-segment in second examination, a display unit which displays the images, an input unit which inputs adjustment information on at least one of respective brightness and respective contrast of the images, and a control unit which controls to switch displays between the images in accordance with switching between the examinations to adjust at least one of the brightness and contrast of the displayed image based on the information.

## Description

### BACKGROUND OF THE INVENTION

### 1. Field of the Invention

The present invention relates to an ophthalmic apparatus for examining (including measuring, photographing and the like) different eye characteristics of an eye of an examinee.

### 2. Description of Related Art

Conventionally, a multifunction ophthalmic apparatus capable of examining different eye characteristics of an eye of an examinee by a single apparatus is proposed. In this kind of apparatus, an observation optical system having an image-pickup element for picking up an image of an anterior-segment of the eye and a display unit including a monitor for displaying the picked-up image are arranged, so that, at the time of examinations of the respective eye characteristics, alignment of the apparatus with respect to the eye and the like are performed while the image of the anterior-segment is observed.

In the examinations of the respective eye characteristics, there is a case where constitutions of the observation optical system and an alignment optical system used therefor are different. In such a case, brightness and contrast of the images of the anterior-segment displayed on the monitor may be different in the respective examinations, and it takes a lot of trouble to adjust them every time the examination is performed.

### SUMMARY OF THE INVENTION

An object of the invention is to overcome the problems described above and to provide an ophthalmic apparatus capable of observing images of an eye of an examinee (images of an anterior-segment of the eye) suitable for respective examinations of different eye characteristics.

To achieve the objects and in accordance with the purpose of the present invention, an ophthalmic apparatus has a first examination part including a first examination optical system for examining a first eye characteristic of an eye of an examinee, a second examination part including a second examination optical system for examining a second eye characteristic of the eye different from the first eye characteristic, a first image-pickup element which picks up a first image of an anterior-segment of the eye at the time of first examination by the first examination part, a second image-pickup element which picks up a second image of the anterior-segment at the time of second examination by the second examination part, a display unit which displays the first image and the second image, an input unit which inputs adjustment information on at least one of respective brightness and respective contrast of the first image and the second image, and a control unit which controls to switch displays between the first image and the second image in accordance with switching between the first examination and the second examination so as to adjust at least one of the brightness and the contrast of the displayed image based on the inputted adjustment information.

Additional objects and advantages of the invention are set forth in the description which follows, are obvious from the description, or may be learned by practicing the invention. The objects and advantages of the invention may be realized and attained by the ophthalmic apparatus in the claims.

### BRIEF DESCRIPTION OF THE DRAWINGS

The accompanying drawings, which are incorporated in and constitute a part of this specification, illustrate embodiments of the present invention and, together with the description, serve to explain the objects, advantages and principles of the invention. In the drawings,
Fig. 1 is a view showing a schematic configuration of an ophthalmic apparatus consistent with the present invention;
Figs. 2A and 2B are views showing a schematic configuration of an inside of a main body when viewed from the side;
Fig. 3 is a view showing a schematic configuration of optical systems arranged inside the main body and an air blowing mechanism in an intraocular pressure measurement part;
Fig. 4 is a view showing a schematic block diagram of a control system of the present apparatus;
Fig. 5 is a view showing an example of an environment setting screen in an eye refractive power measurement mode and a corneal shape measurement mode; and
Fig. 6 is a view showing a schematic configuration of a partially modified embodiment of the optical systems arranged inside the main body.

### DETAILED DESCRIPTION OF THE PREFERRED EMBODIMENTS

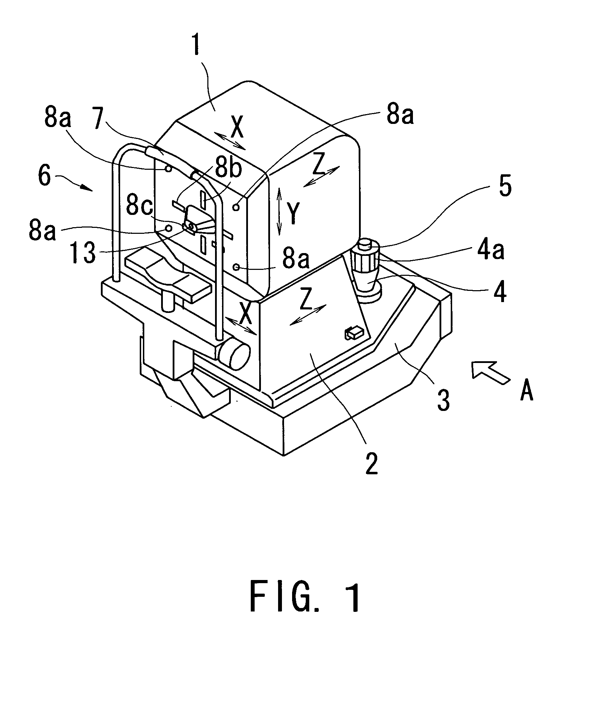

A detailed description of one preferred embodiment of an ophthalmic apparatus embodied by the present invention is provided below with reference to the accompanying drawings. In this embodiment, a multifunction apparatus which measures intraocular pressure, eye refractive power and a corneal shape will be taken as an example. Fig. 1 is a view showing a schematic configuration of an ophthalmic apparatus consistent with the present invention.

A mobile base 2, on which a main body 1 is provided, is arranged on a base 3 movably in a right/left direction (referred to as an X-direction hereinafter) and a back/forth direction (a working distance direction, referred to as a Z-direction hereinafter). This movement is mechanically (or may be electrically) performed through operation of a joystick 4. Additionally, the main body 1 is arranged on the mobile base 2 movably in the X-direction, Z-direction, and an up/down direction (referred to as a Y-direction hereinafter). The movement in each of the directions is electrically performed based on a detection result on an alignment state of the main body 1 with an eye of an examinee. Further, the movement in the Y-direction is electrically performed also through operation of a rotation knob 4a of the joystick 4.

On the base 3, a face-supporting unit 6 for supporting a face (a head) of the examinee is fixedly provided. Further, a forehead rest 7, against which a forehead of the examinee is slightly pressed to be fixed, is provided to the face-supporting unit 6. A measurement starting switch 5 is provided at the top of the joystick 4. Projection windows 8a allow the passage of light from light sources 20 for anterior-segment illumination described later. Projection windows 8b allow the passage of light from light sources 80 for corneal shape measurement and alignment in the Z-direction described later. Projection windows 8c allow the passage of light from light sources 85 for the alignment in the Z-direction described later.

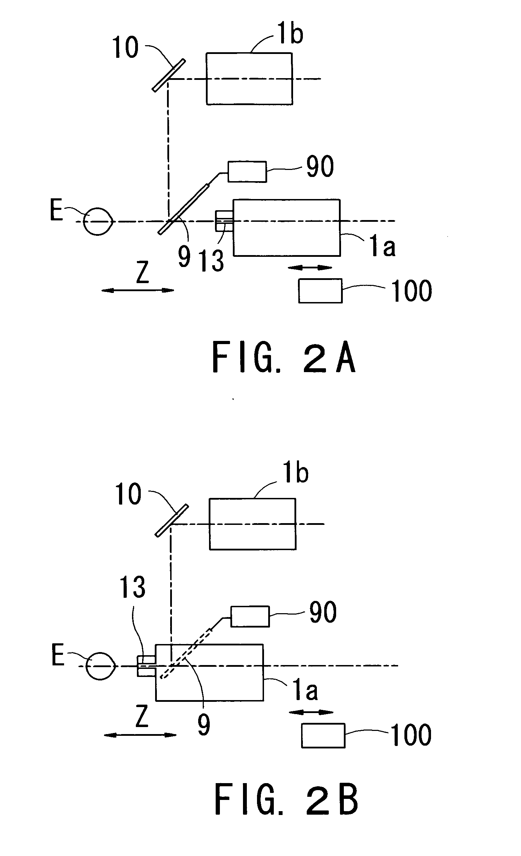

Figs. 2A and 2B are views showing a schematic configuration of an inside of the main body 1 when viewed from the side (i.e., from a direction of the arrow A shown in Fig. 1). Inside the main body 1, an intraocular pressure measurement part 1a for performing noncontact measurement of the intraocular pressure of an eye E of the examinee is arranged movably in the Z-direction, and an eye refractive power/corneal shape measurement part 1b for measuring the eye refractive power and the corneal shape of the eye E is fixedly arranged above the measurement part 1a. Further, a reflection mirror 9, a reflection mirror 10, a mirror moving unit 90, and a moving unit 100 for the measurement part 1a are arranged.

The measurement part 1a is moved in parallel in the Z-direction by the moving unit 100. The moving unit 90 performs insertion and removal of the mirror 9 between the eye E and a nozzle 13 provided to the measurement part 1a in synchronization with the movement of the measurement part 1a in the Z-direction. In other words, when the measurement part 1a is moved from a retreat position in Fig. 2A to a measurement reference position in Fig. 2B, the mirror 9 is moved from a state where the mirror 9 is inserted in front of the nozzle 13 (i.e., between the eye E and the nozzle 13) to a state where the mirror 9 is removed therefrom. In contrast, when the measurement part 1a is moved from the measurement reference position in Fig. 2B to the retreat position in Fig. 2A, the mirror 9 is also moved from the removed state to the inserted state.

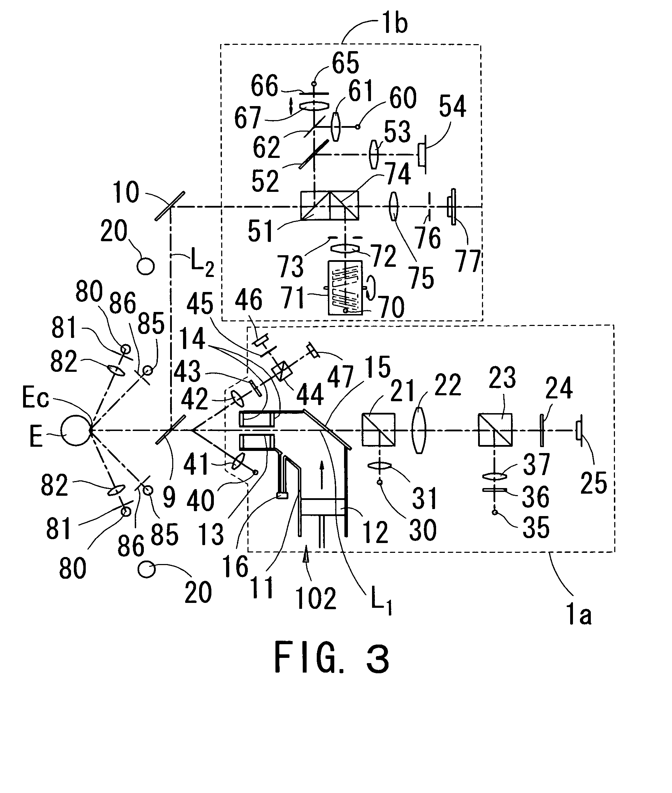

Fig. 3 is a view showing a schematic configuration of optical systems arranged inside the main body 1 and an air blowing mechanism in the measurement part 1a.

Firstly, an air (fluid) blowing mechanism 102 in the measurement part 1a will be described. A cylinder 11 is for air compression. A piston 12 is moved inside the cylinder 11 by driving force of a rotary solenoid 113 described later. The air compressed inside the cylinder 11 by the movement of the piston 12 is blown from the nozzle 13 to a cornea Ec of the eye E. Two transparent glass plates 14 hold the nozzle 13. A transparent glass plate 15 is provided behind the nozzle 13. Behind the glass plate 15, optical systems for observation and alignment described later are arranged. A pressure sensor 16 detects pressure inside the cylinder 11.

Next, the optical systems in the measurement part 1a will be described. In this regard, when using the measurement part 1a (i.e., at the time of intraocular pressure measurement) , the mirror 9 is moved by the moving units 90 and 100 to the retreat position (removed state) where it does not influence the intraocular pressure measurement.

Four infrared light sources 20 for anterior-segment illumination are arranged having an optical axis L1 coincident with an axial line of the nozzle 13 as their center. An image of an anterior-segment of the eye E formed by the light sources 20 is transmitted through the glass plate 15, a half mirror 21, an objective lens 22, a dichroic mirror 23, and a filter 24, each arranged on the optical axis L1, to be picked up by a CCD camera 25 being an image-pickup element (these components constitute an observation optical system). Besides, the dichroic mirror 23 has a property of transmitting infrared light and reflecting visible light. Additionally, the filter 24 has a property of transmitting light from the light sources 20 and a light source 30 described later, and not transmitting light from a light source 40 described later. The image of the anterior-segment of the eye E picked up by the camera 25 is displayed on a monitor 26 described later. Besides, the light sources 20 are configured so that light intensity thereof is adjustable.

The infrared light source 30 is for alignment in the X-and Y-directions, and the light therefrom is transmitted through a projection lens 31, the half mirror 21, and the glass plate 15, to be projected onto the cornea Ec from the front (these components constitute an alignment target projection optical system). An image of corneal reflection by the light source 30 (corneal reflex of the light source 30) is transmitted through the glass plate 15 to the filter 24 to be picked up by the camera 25 (these components constitute an alignment target detection optical system), and is utilized in the alignment in the X-and Y-directions. Besides, images of corneal reflection by the light sources 20 (corneal reflexes of the light sources 20) may also be utilized in the alignment in the X-and Y-directions (for details, see US 6,022,108 corresponding to Japanese Patent Application Unexamined Publication No. Hei10-71122). A visible light source 35 is for fixation target projection, and light from a fixation target 36 illuminated by the light source 35 is transmitted through a projection lens 37, the dichroic mirror 23, the objective lens 22, the half mirror 21, and the glass plate 15, to head for the eye E.

The infrared light source 40 is for detection of a deformation state of the cornea Ec, and the light therefrom is made into an approximately parallel light bundle by a collimator lens 41 to be projected onto the cornea Ec. An image of corneal reflection by the light source 40 (corneal reflex of the light source 40) is transmitted through a photo-receiving lens 42, a filter 43, a half mirror 44, and a pinhole plate 45, to be photo-received on a photo-detector 46. The filter 43 has a property of transmitting the light from the light source 40 and not transmitting the light from the light sources 20 and 30. A corneal-deformation-state detection optical system (an intraocular pressure measurement optical system) constituted of these components is arranged such that a photo-receiving amount of the photo-detector 46 is at the maximum when the cornea Ec is in a predetermined deformation state (a flat state).

In addition, the light source 40 and the collimator lens 41 are also utilized in alignment in the Z-direction (these components constitute an alignment target projection optical system), and the image of corneal reflection by the light source 40 is transmitted through the photo-receiving lens 42 to the half mirror 44 and enters a one-dimensional position detector 47 such as a PSD or a line sensor (these components constitute an alignment target detection optical system). When the eye E (cornea Ec) is moved in the Z-direction, an entering position of the image of corneal reflection by the light source 40 is also moved on the position detector 47. Therefore, an alignment state in the Z-direction with respect to the eye E may be detected based on an output signal from the position detector 47.

Incidentally, for the sake of illustration, Fig. 3 shows such that the optical system for detection of a deformation state of a cornea and the optical system for detection of the alignment state in the Z-direction are vertically arranged; however, they are primarily arranged horizontally with respect to the eye of the examinee.

Next, optical systems in the measurement part 1b will be described. When the measurement part 1b is used (i.e., at the time of eye refractive power measurement and corneal shape measurement), the mirror 9 is moved by the moving units 90 and 100 to the measurement reference position (inserted state) in front of the nozzle 13.

The image of the anterior-segment of the eye E formed by the light sources 20 is reflected by the mirror 9, and is transmitted through the mirror 10, a half mirror 51, a half mirror 52, and an image forming lens 53, each arranged on a measurement optical axis L2 made coaxial with the optical axis L1 by the mirror 9, to be picked up by a CCD camera 54 being an image-pickup element (these components constitute an observation optical system). The image of the anterior-segment of the eye E picked up by the camera 54 is displayed on the monitor 26 described later.

An infrared light source 60 is for the alignment in the X-and Y-directions, and light theref rom is transmitted through a projection lens 61, a dichroic mirror 62, the half mirror 52, the half mirror 51, the mirror 10 and the mirror 9, to be projected onto the cornea Ec from the front (these components constitute an alignment target projection optical system). Incidentally, the dichroic mirror 62 has a property of transmitting visible light and reflecting infrared light. An image of corneal reflection by the light source 60 (corneal reflex of the light source 60) is transmitted through the mirror 9 to the image forming lens 53 to be picked up by the camera 54 (these components constitute an alignment target detection optical system), and is utilized in the alignment in the X-and Y-directions. Besides, the images of corneal reflection by the light sources 20 may also be utilized in the alignment in the X-and Y-directions. Avisible light source 65 is for fixation target projection, and light from a fixation target 66 illuminated by the light source 65 is transmitted through a projection lens 67, the dichroic mirror 62, the half mirror 52, the half mirror 51, the mirror 10 and the mirror 9 to head for the eye E. In addition, the projection lens 67 is moved in the direction of an optical axis, so that the eye E is fogged.

An infrared light source 70 is for eye refractive power measurement, and light therefrom passes through slits provided in a rotation sector 71, and is transmitted through a projection lens 72, a diaphragm 73, a half mirror 74, the half mirror 51, the mirror 10, and the mirror 9, to be projected onto a fundus of the eye E while being scanned. The light reflected from the fundus is transmitted through the mirror 9, the mirror 10, the half mirror 51, the half mirror 74, a photo-receiving lens 75 and a diaphragm 76, and is photo-received on a photo-receiving part 77 provided with a plurality of pairs of photodetectors. Incidentally, in connection with the optical system for the eye refractive power measurement, see US 5,907,388 corresponding to Japanese Patent Application Unexamined Publication No. Hei10-108836 for details.

Four infrared light sources 80 for the corneal shape measurement and the alignment in the Z-direction are arranged having the optical axis L1 as their center. Two of them are arranged in a horizontal direction of the apparatus, and the other two are arranged in a vertical direction of the apparatus, in order that each of their projection optical axes intersects at a predetermined angle with the optical axis L1. Light from the light sources 80 is transmitted through spot diaphragms 81 and collimating lenses 82 to be projected onto the cornea Ec (these components constitute an alignment target projection optical system). Images of corneal reflection by the light sources 80 (corneal reflexes of the light sources 80) are transmitted through the mirror 9, the mirror 10, the half mirror 51, the half mirror 52, and the image forming lens 53, to be picked up by the camera 54 (these components constitute an alignment target detection optical system). Besides, for details of the optical system for the corneal shape measurement, see Japanese Patent Application Unexamined Publication No. Sho61-85920.

Two infrared light sources 85 for the alignment in the Z-direction are arranged having the optical axis L1 as their center. Further, the light sources 85 are arranged in the horizontal direction of the apparatus so that each of their projection optical axes intersects at a predetermined angle with the optical axis L1. Light from the light sources 85 is transmitted through spot diaphragms 86 to be projected onto the cornea Ec (these components constitute an alignment target projection optical system). Images of corneal reflection by the light sources 85 (corneal reflexes of the light sources 85) are transmitted through the mirror 9, the mirror 10, the half mirror 51, the half mirror 52, and the image forming lens 53, to be picked up by the camera 54.

Since the light from the light sources 80 is made into a parallel light bundle by the lenses 82, even if a working distance (distance in the Z-direction) of the main body 1 with respect to the eye E is changed, a position of the image of corneal reflection is changed little. In contrast, since the light from the light sources 85 is a divergent light bundle, if the working distance is changed, a position of the image of corneal reflection is changed. Thus, an alignment state in the Z-direction may be detected from the positions of these images of corneal reflection (for details, see US 5,463,430 corresponding to Japanese Patent Application Unexamined Publication No. Hei6-46999).

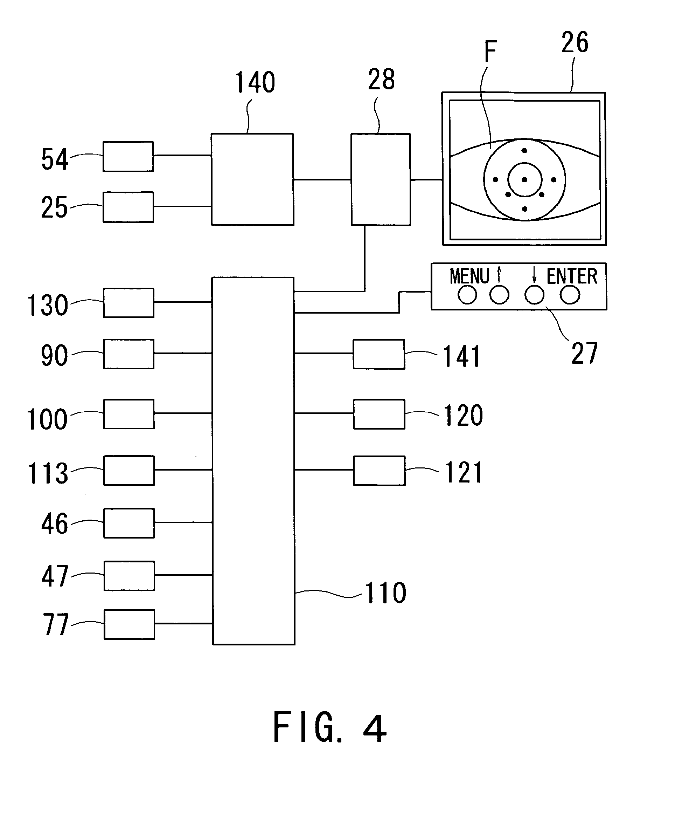

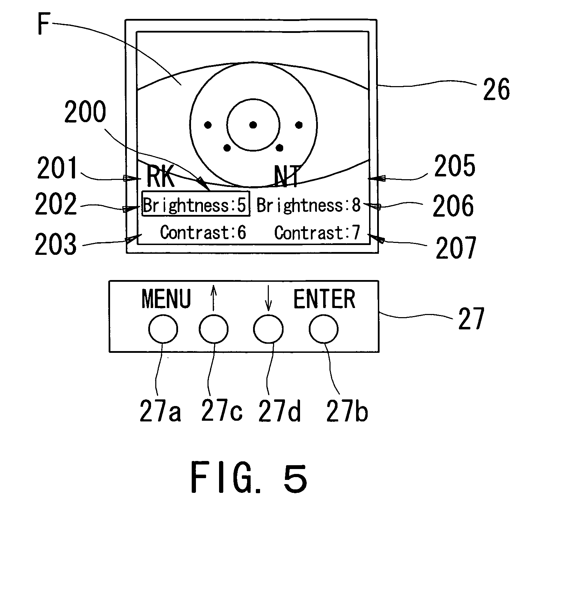

Fig. 4 is a view showing a schematic block diagram of a control system of the present apparatus. Inputted into an image control part 140 are image signals from the camera 25 and the camera 54. The image control part 140 has functions such as controlling to process the image signals to detect the images of corneal reflection by the respective light sources, to switch the image signals to be outputted on the monitor 26 (display images on the monitor 26), and to superimpose letters, marks and the like on the image of the anterior-segment of the eye E. A display setting part 27 is for inputting adjustment information on brightness and contrast of the display screen (the display image) on the monitor 26, and the adjustment information is inputted into a system control part 110. The display setting part 27 is provided with a MENU button 27a, an ENTER button 27b, an up (↑) button 27c, and a down (↓) button 27d (see Fig. 5). A monitor control part 28 controls to adjust and set the brightness and the contrast of the monitor 26 based on an instruction signal from the system control part 110. For the monitor control part 28, an adjustment function provided to the monitor 26 may also be utilized.

The image control part 140 is connected with the monitor control part 28 which is connected to the system control part 110. In addition, the system control part 110 is connected with the likes of the display setting part 27, a memory 141 for storing an adjustment value of the monitor 26 in digital form, a moving part 130 for moving the main body 1 three-dimensionally in the X-, Y-and Z-directions, the moving unit 90, the moving unit 100, the rotary solenoid 113 for moving the piston 12, the photo-detector 46, the position detector 47, the photo-receiving part 77, a memory 120 for storing measurement data, measurement conditions and the like, and a switch part 121 having a measurement-mode selecting switch, a light-intensity adjustment switch for adjusting the light intensity of the light sources 20 and the like.

In the ophthalmic apparatus having the aforementioned constitution, its operations will be described. The present apparatus includes a first mode; the apparatus operates only in an eye refractive power measurement mode, a second mode; the apparatus operates only in a corneal shape measurement mode, a third mode; the apparatus operates only in an intraocular pressure measurement mode, a forth mode; the apparatus operates successively in the eye refractive power measurement mode and the corneal shape measurement mode, and a fifth mode; the apparatus operates successively in the eye refractive power measurement mode, the corneal shape measurement mode and the intraocular pressure measurement mode. These modes may be selected by the mode selecting switch in the switch part 121. In the fifth mode, the apparatus firstly operates in the eye refractive power measurement mode and the corneal shape measurement mode, and then, it operates in the intraocular pressure measurement mode upon automatic switching to the intraocular pressure measurement mode. This is because, if the intraocular pressure is measured first, an influence of the blow of the compressed air and the like possibly remains. Hereinafter, the fifth mode will be described.

In the eye refractive power measurement mode and the corneal shape measurement mode, the mirror 9 is inserted in front of the nozzle 13 and the image of the anterior-segment of the eye E is picked up by the camera 54. The examiner performs rough alignment in the respective X-, Y- and Z-directions of the main body 1 with the eye E through operation of the joystick 4 and the rotation knob 4a while observing the image of the anterior-segment displayed on the monitor 26.

When each of the images of corneal reflection by the light sources 60, 80 and 85 is in a condition to be picked up by the camera 54, the images are detected to be processed by the image control part 140. The system control part 110 drives and controls the moving part 130 based on detection results on the images, and performs detailed (fine) alignment in the X-, Y-and Z-directions of the main body 1 with the eye E.

Once the alignment state in each of the X-, Y-and Z-directions with the eye E falls respectively within a predetermined permissible range, the system control part 110 automatically controls to emit a trigger signal (or the examiner depresses the switch 5 to input the trigger signal in accordance with a display of alignment completion displayed on the monitor 26), and the eye refractive power is obtained based on phase differences of output signals from the respective photodetectors in the photo-receiving part 77. In addition, the system control part 110 controls to obtain a position of the image of corneal reflection by the light sources 80 and calculate the corneal shape, based on an output signal from the camera 54.

In the fifth mode, when the eye refractive power measurement and the corneal shape measurement satisfy a predetermined condition for measurement termination such that the predetermined number of measurement results are obtained respectively, the system control part 110 automatically controls to emit a switching signal to the intraocular pressure measurement mode, and the eye refractive power measurement mode and the corneal shape measurement mode are switched to the intraocular pressure measurement mode. Based on the switching signal to the intraocular pressure measurement mode, the system control part 110 controls to turn off the light sources 60, 80, 85 and the like for the alignment in the eye refractive power measurement and the corneal shape measurement, and turn on the light sources 30, 40 and the like for the alignment of the intraocular pressure measurement. At the same time, the system control part 110 controls to move the measurement part 1a forward (in a direction toward the eye E), and thrust a tip of the nozzle 13 from a front surface of the main body 1. At this time, the mirror 9 is removed from the front of the nozzle 13 in conjunction with the movement of the measurement part 1a, and thereby it becomes possible to pick up the image of the anterior-segment of the eye E by the camera 25, and each of the images of corneal reflection by the light sources 30 and 40 is brought in a detectable state. Further, the system control part 110 controls to switch the images displayed on the monitor 26 from the one picked up by the camera 54 to the one picked up by the camera 25 based on the switching signal to the intraocular pressure measurement mode. The examiner, if necessary, manually performs rough alignment and the like while observing the image of the anterior-segment displayed on the monitor 26.

When the image of corneal reflection by the light source 40 is in a state of entering the position detector 47, the system control part 110 drives and controls the moving part 130 (or the moving unit 100, or both of the moving part 130 and the moving unit 100) based on a detection result on the image, and performs detailed (fine) alignment in the z-direction. Further, the moving part 130 is driven and controlled based on a detection result on the image of corneal reflection by the light source 30, which is picked up by the camera 25, to perform detailed (fine) alignment in the X-and Y-directions.

Once an alignment state in each of the X-, Y-and Z-directions of the measurement part 1a with respect to the eye E falls respectively within a predetermined permissible range, the system control part 110 automatically controls to emit the trigger signal (or the examiner depresses the switch 5 to input the trigger signal in accordance with the display of alignment completion displayed on the monitor 26), and to drive the rotary solenoid 113. When the piston 12 is moved by the driving of the rotary solenoid 113, the air inside the cylinder 11 is compressed and blown from the nozzle 13 to the cornea Ec. The cornea Ec is gradually deformed by the blow of the compressed air, and when it reaches the flat state, the maximum amount of light enters the photo-detector 46. The system control part 110 controls to calculate the intraocular pressure based on an output signal from the pressure sensor 16 and an output signal from the photo-detector 46.

Next, described will be a method for adjusting the brightness and the contrast of the image of the anterior-segment displayed on the monitor 26, when those at the time of the eye refractive power measurement and the corneal shape measurement differ from those at the time of the intraocular pressure measurement.

By depressing the button 27a on the display setting part 27, an environment setting screen is displayed on the monitor 26. Fig. 5 is a view showing an example of the environment setting screen in the eye refractive power measurement mode and the corneal shape measurement mode, where an image F of the anterior-segment picked up by the camera 54 is displayed on the monitor 26. At the left side of the lower part of the screen, displayed are an "RK" display 201 indicating the eye refractive power measurement mode and the corneal shape measurement mode, and a display 202 of an adjustment level of the brightness and a display 203 of an adjustment level of the contrast in these modes. At the right side of the lower part of the screen, displayed are an "NT" display 205 indicating the intraocular pressure measurement mode, and a display 206 of an adjustment level of the brightness and a display 207 of an adjustment level of the contrast in this mode. The adjustment level of each parameter of the brightness and the contrast is adjustable, for example, in 10 levels from 1 to 10. A cursor 200 indicates a selected item among the parameters.

Here, in a case where the brightness and the contrast on the monitor 26 in the eye refractive power measurement mode and the corneal shape measurement mode are adjusted, the button 27c or the button 27d is depressed to move the cursor 200 to the parameter to be adjusted, and the button 27b is depressed to select the parameter, so that the adjustment level thereof becomes changeable. Then, by depressing the button 27c or the button 27d again, the adjustment level of the selected parameter may be increased or decreased. An operation signal from the display setting part 27 is inputted into the system control part 110, from which the instruction signal is transmitted to the monitor control part 28, and thereby the brightness and the contrast on the monitor 26 are adjusted. At the time of the adjustment, the brightness and the contrast may be adjusted to be brought in a state desired by the examiner through observation of the displayed image F of the anterior-segment.

When the button 27a is depressed after the adjustment of the brightness and the contrast on the monitor 26, the screen on the monitor 26 is switched to an original screen, and the respective adjustment values in the eye refractive power measurement mode and the corneal shape measurement mode are stored in the memory 141. Besides, the memory 141 is a non-volatile memory which maintains its memory even after power supply of the apparatus is shut off.

In the same way as is described, when the button 27a on the display setting part 27 is depressed after switching to the intraocular pressure measurement mode, an environment setting screen in the intraocular pressure measurement mode is displayed on the monitor 26. Incidentally, at the time of the switching to the intraocular pressure measurement mode, the measurement part 1a is moved to an eye E side and the mirror 9 is removed from the front of the nozzle 13, so that the image of the anterior-segment of the eye E is picked up by the camera 25, as described above. Also in this case, the button 27c or the button 27d is depressed to move the cursor 200 to the parameter to be adjusted, and the button 27b is depressed to select the parameter. Then, by depressing the button 27c or the button 27d again, the adjustment level of the selected parameter may be increased or decreased, so that the brightness and the contrast of the image F of the anterior-segment displayed on the monitor 26 may be adjusted to be brought in a desired state. And then, by depressing the button 27a, the screen on the monitor 26 is switched to an original screen, and the respective adjustment values in the intraocular pressure measurement mode are stored in the memory 141.

Incidentally, it is preferable that the brightness and the contrast are adjusted while checking the respective images F of the anterior-segment picked up by the cameras 25 and 54, but a model eye may substitute for the eye E for the check.

After the adjustment as described above is performed, according to the signals for switching to the eye refractive power measurement mode, the corneal shape measurement mode and the intraocular pressure measurement mode, the system control part 110 controls to read from the memory 141 the respective adjustment values of the brightness and the contrast corresponding to the respective modes, and input the instruction signals based on the adjustment values into the monitor control part 28. The monitor control part 28 controls to adjust and set the brightness and the contrast based on the inputted instruction signals, and thereby the examiner may easily observe the images of the anterior-segment suitable for the respective modes. If the images of the anterior-segment in the eye refractive power measurement mode, the corneal shape measurement mode and the intraocular pressure measurement mode are brought in a state where they may be observed under the similar brightness and the similar contrast, an uncomfortable feeling caused when the modes are switched is lessen, so that the images of the anterior-segment are easily observed at the time of the respective measurements.

Incidentally, a constitution may be employed, where a control circuit for adjusting respective gains of the image signals outputted from the cameras 25 and 54 is provided instead of the monitor control part 28.

Further, as a modified embodiment for adjusting the brightness of the image of the anterior-segment, a constitution may be employed where the light intensity of the light sources 20 is adjusted. In this case, intensity of illumination light in the respective modes is adjusted by a light-intensity adjustment switch in the switch part 121, and its adjustment values are stored in the memory 141. In the same way as is described, according to the signals for switching to the respective modes, the system control part 110 controls to read the adjustment values of the light intensity corresponding to the respective modes, and to adjust the light intensity of the light sources 20. Light-intensity adjustment as such may be performed not only by adjusting luminescence intensity of the light sources themselves, but also by adjusting the number of the light sources to light.

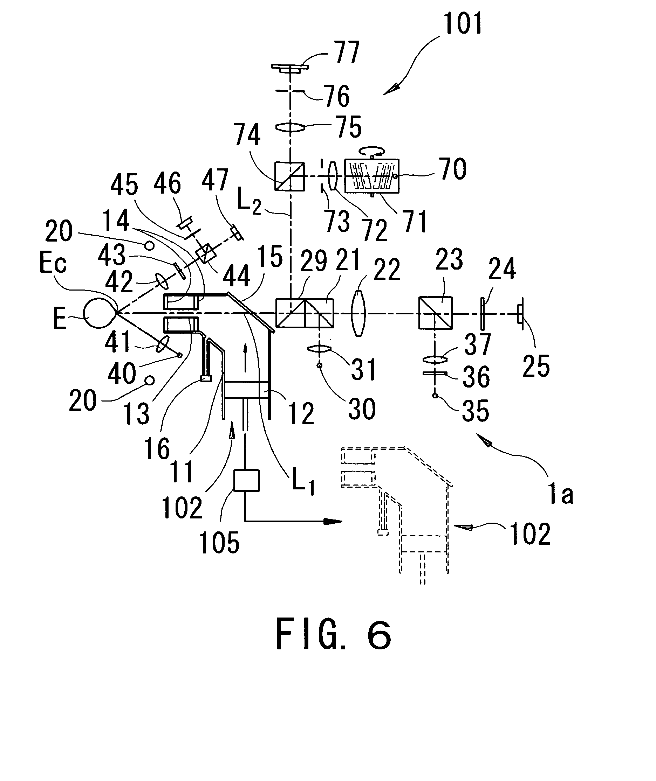

Fig. 6 is a schematic configuration of a partially modified embodiment of the optical systems arranged inside the main body 1. Components having the same numeral references as those in Fig. 3 have basically the same constitutions. In the modified embodiment, a constitution is employed, where a part of the observation optical system including the camera 25 in the measurement part 1a is used also at the time of the eye refractive power measurement. In the eye refractive power measurement mode, the air blowing mechanism 102 is removed from the optical axis L1 by a moving mechanism 105 so that measurement light is not shaded by the air blowing mechanism 102. An eye refractive power measurement optical system 101 is disposed on an optical path bifurcated by a beam splitter 29. At the time of the eye refractive power measurement, the eye E is fogged by moving the fixation target 36. The constitution is such that the light sources for alignment is used also at the time of the eye refractive power measurement; however, dedicated light sources may be prepared instead. The respective alignment states at the time of the eye refractive power measurement and the intraocular pressure measurement are detected by the camera 25 used in both the measurements.

In this constitution, as the air blowing mechanism 102 is disposed on the optical axis L1 at the time of the intraocular pressure measurement, the observation optical system to the camera 25 partly differs from that at the time of the eye refractive power measurement. In other words, at the time of the intraocular pressure measurement, the nozzle 13 included in the air blowing mechanism 102, the two glass plates 14 holding the nozzle 13, and the glass plate 15 behind them are disposed. The glass plate 14 holding the nozzle 13 arranged at the front end is usually in a size of the order of 10 mm to avoid contact of the nozzle 13 with a face part around the eye E. In contrast, at the time of the eye refractive power measurement, the optical system included in the air blowing mechanism 102 is removed from the optical axis L1, so that a measurement window for eye refractive power measurement may take up a larger space. In such a case, the brightness of the image of the anterior-segment picked up by the camera 25 at the time of the intraocular pressure measurement and displayed on the monitor 26 is darker than that at the time of the eye refractive power measurement; however, if the brightness and the contrast are respectively adjusted at the time of the eye refractive power measurement and the intraocular pressure measurement as described above, the images of the anterior-segment become easy to be observed at the time of the respective measurements.

The above description has been made taking the multifunction apparatus for performing the intraocular pressure measurement, the eye refractive power measurement and the corneal shape measurement as an example; however, the present invention may be applied to other multifunction apparatuses which perform various kinds of examination, measurement, photographing and the like, such as a multifunction apparatus for performing the intraocular pressure measurement and fundus photographing, and a multifunction apparatus for performing the eye refractive power measurement and sectional photographing of the anterior-segment of the eye.

The foregoing description of the preferred embodiments of the invention has been presented for purposes of illustration and description. It is not intended to be exhaustive or to limit the invention to the precise form disclosed, and modifications and variations are possible in the light of the above teachings or may be acquired from practice of the invention. The embodiments chosen and described in order to explain the principles of the invention and its practical application to enable one skilled in the art to utilize the invention in various embodiments and with various modifications as are suited to the particular use contemplated. It is intended that the scope of the invention be defined by the claims appended hereto.

## Claims

1. An ophthalmic apparatus comprising:
a first examination part including a first examination optical system for examining a first eye characteristic of an eye of an examinee;
a second examination part including a second examination optical system for examining a second eye characteristic of the eye different from the first eye characteristic;
a first image-pickup element which picks up a first image of an anterior-segment of the eye at the time of first examination by the first examination part;
a second image-pickup element which picks up a second image of the anterior-segment at the time of second examination by the second examination part;
a display unit which displays the first image and the second image;
an input unit which inputs adjustment information on at least one of respective brightness and respective contrast of the first image and the second image; and
a control unit which controls to switch displays between the first image and the second image in accordance with switching between the first examination and the second examination so as to adjust at least one of the brightness and the contrast of the displayed image based on the inputted adjustment information.

2. The ophthalmic apparatus according to claim 1, further comprising an illumination light source which illuminates the anterior-segment of the eye,
wherein the control unit controls to adjust light intensity of the illumination light source so as to adjust the brightness of the displayed image.

3. The ophthalmic apparatus according to claim 1, wherein the first image-pickup element doubles as the second image-pickup element.

4. The ophthalmic apparatus according to claim 1, further comprising:
a first alignment target projection optical system for projecting a first alignment target onto the eye, the first alignment target being for aligning the first examination part with the eye; and
a second alignment target projection optical system for projecting a second alignment target onto the eye, the second alignment target being for aligning the second examination part with the eye.

5. The ophthalmic apparatus according to claim 1,
wherein the first eye characteristic includes at least one of eye refractive power and a corneal shape, and
the second eye characteristic includes intraocular pressure.
